# FASCICULE DE BREVET EUROPEEN

(11) **EP 3 866 855 B1**
(45) Date de publication et mention de la délivrance du brevet: **09.12.2026**
(21) Numéro de dépôt: 19806030.3
(22) Date de dépôt: 16.10.2019
(51) Int. Cl.: A61K 47/44, C08L 91/06, A23K 10/00

(54) **COMPOSITION LIPIDIQUE POUR L'ENCAPSULATION D'UNE SUBSTANCE ACTIVE ET PERMETTANT LE CONTRÔLE DE LA VITESSE DE LIBÉRATION DE LADITE SUBSTANCE ACTIVE**
LIPIDZUSAMMENSETZUNG ZUR VERKAPSELUNG EINES WIRKSTOFFS ZUR STEUERUNG DER FREISETZUNGSRATE DES BESAGTEN WIRKSTOFFS
LIPID COMPOSITION FOR ENCAPSULATING AN ACTIVE SUBSTANCE, PERMITTING CONTROL OF THE RATE OF RELEASE OF SAID ACTIVE SUBSTANCE

(30) Priorité: 19.10.2018 FR 1859696
(43) Date de publication de la demande: 25.08.2021
(60) Demande divisionnaire: 21211618.0 / 3 995 151
(73) Titulaire: Société d'Exploitation de Produits pour les Industries Chimiques SEPPIC, 75321 Paris cedex 07 (FR)
(72) Inventeur: FAGET, Sophie, 81100 CASTRES (FR); LEFEBVRE, Sandra, 81100 CASTRES (FR)
(74) Mandataire: Air Liquide
(86) Numéro de dépôt international: PCT/FR2019/052454
(87) Numéro de publication internationale: WO 2020/079367

(56) Documents cités:
- WO-A1-91/05548

## Description

La présente invention est relative à une composition lipidique, à une composition à libération contrôlée comprenant ladite composition lipidique et permettant le contrôle de la vitesse de libération de la substance active qu'elle contient, et à un procédé de fabrication de la formulation galénique comprenant la composition à libération contrôlée.

Les principes actifs développés et mis en œuvre, dans les formulations pharmaceutiques, humaines et vétérinaires, dans les formulations des industries de compléments alimentaires sont sensibles aux facteurs environnementaux, que ce soit lors de la fabrication des formes galéniques associées (notamment aux températures élevées pratiquées dans certains procédés, aux phénomènes d'oxydation....), et/ou lors de la durée de vie desdites formes galéniques, et/ou lors de la consommation, dans l'organisme humain ou animal, lors de la mise en contact desdites formes galéniques avec les molécules de dégradation et/ou de digestion présentes dans les organismes des consommateurs.

Par ailleurs, les molécules constituant en toute ou partie les principes actifs présents dans ces formes galéniques, peuvent présenter des caractéristiques organoleptiques non compatibles avec une consommation directe : un mauvais goût, une odeur non pertinente...

Pour apporter les avantages nutritionnels ou thérapeutiques visés, les molécules introduites dans des capsules, des gélules, des comprimés voire dans de la nourriture ne doivent pas se dissoudre à la même vitesse et dans le même environnement.

C'est notamment pour ces différentes raisons que de nombreuses molécules actives sont impliquées dans un procédé dit d'encapsulation, dont l'objectif est de préparer une composition (C_{A}) comprenant au moins un principe actif. Ce procédé d'encapsulation a pour fonction de protéger l'au moins principe actif d'interactions non souhaitées avec un milieu extérieur spécifique, et de le transporter jusque dans un autre milieu dans lequel les conditions permettront une libération de l'actif pour accomplir sa fonction thérapeutique ou nutritionnelle prévue. Ce processus est un cas particulier du processus nommé « fonctionnalisation directe » par l'homme du métier.

Par « fonctionnalisation directe », on entend la modification d'un moins une propriété physico chimique de l'API par la mise en œuvre d'une formulation particulière et au moyen de procédés spécifiques.

Parmi les technologies d'encapsulation les plus connues, on peut citer les technologies mettant en jeu des composés majoritairement hydrophiles mais également, les technologies mettant en jeu des composés majoritairement hydrophobes comme par exemple les technologies dites de « prilling » à savoir les technologies comprenant une étape d'atomisation d'une forme lipidique contenant au moins un actif de façon à obtenir une forme sphérique solide, aussi dénommée « Spray Chilling » ou « Spray Cooling», de congélation ou « Congealing », de revêtement en milieu fondu ou « Hot Melt Coating », d'extrusion en milieu fondu ou « Hot Melt Extrusion », de granulation en milieu fondu ou « Melt granulation », de pastillage ou « Pelletisation », de sphéronisation, de granulation thermique ou «thermo-granulation »...

Cette « fonctionnalisation directe », notamment par l'intermédiaire d'une technique d'encapsulation, peut donc présenter comme avantage une stabilisation de certains composés intrinsèquement instables, comme par exemple les composés volatils, une prévention des phénomènes d'oxydation, une protection globale de l'activité médicamenteuse ou nutritionnelle.

Cette étape de « fonctionnalisation directe» initiale, et notamment par l'intermédiaire d'une technique d'encapsulation, peut également avoir un impact sur la cinétique de libération de l'actif dans l'organisme de l'hôte. Cet effet est souvent recherché, notamment dans le but d'un masquage de goût, d'odeur ou afin d'obtenir une libération retardée, contrôlée de l'actif auprès de la zone biologique ciblée dans le corps humain ou animal.

L'impact de cette étape de « fonctionnalisation directe» sur le profil de libération de l'actif est pris en compte lors des phases de développement d'une composition thérapeutique ou prophylactique ou nutritionnelle.

Cependant, lors des phases d'extrapolation d'échelles, au cours desquelles des outils industriels de mises en forme sont utilisés, et lesdits outils impliquant des contraintes mécaniques sur la forme galénique, l'homme du métier se trouve confronté au problème récurrent d'une évolution non souhaitée du profil de libération contrôlé par rapport à la formulation initialement testée à des échelles ne nécessitant pas la mise en œuvre de tels outils.

A titre d'exemple, le profil de libération d'un actif après « fonctionnalisation directe» peut être drastiquement modifié, accéléré ou ralenti, suite à une mise en œuvre dans un processus induisant un stress mécanique important tel qu'un processus de compression. On peut ainsi dénaturer une protection lipidique induite par la « fonctionnalisation » initiale, en fragilisant la "coque protectrice" lors de la compression, ce qui peut induire un profil de libération de l'actif accéléré.

Au cours des étapes avals de mise en forme galénique de ces formulations à libération prolongées, on a pu observer que le profil de libération de l'actif obtenu et mis en évidence après fonctionnalisation, peut être affecté significativement et montrer une dissimilitude importante par rapport au profil de libération de l'actif avant mise en forme galénique. Ainsi, le profil de libération d'un actif après fonctionnalisation peut être drastiquement modifié, accéléré ou ralenti, suite à la mise en œuvre d'une étape faisant intervenir un stress mécanique significatif, dans un procédé de préparation dudit actif fonctionnalisé, comme par exemple une étape de compression. De façon plus particulière à titre d'exemple, on peut dénaturer une protection lipidique induite par la fonctionnalisation initiale, en fragilisant la "coque protectrice" lors de la compression, ce qui peut amener un profil de libération de l'actif accéléré et non désiré.

La demande WO9105548A1 divulgue une composition à libération entretenue permettant d'administrer des protéïnes macromoléculaire à un animal pendant une durée prolongée.

A ce jour et à notre connaissance, l'homme du métier ne dispose pas de solution permettant de mettre au point et de fabriquer des formulations, comprenant un principe actif à libérer de façon retardée ou prolongée, et dont le profil de libération n'est pas altéré par un procédé de préparation comprenant au moins une étape mettant en œuvre un stress mécanique significatif sur ladite formulation et/ou sur une forme galénique préparée selon un procédé comprenant au moins une étape mettant en œuvre un stress mécanique significatif.

Une solution de la présente invention est une composition lipidique comprenant pour 100% de sa masse :
- de 40% à 99,9% massique, plus particulièrement de 40% à 98%, et encore plus particulièrement de 40% à 95% massique d'un ensemble comprenant pour 100% de sa masse de 90% à 100% massique de cire d'abeille et jusqu'à 10% massique d'au moins un autre excipient lipidique,
- de 0,1% à 60% massique, plus particulièrement de 2% à 60% massique, et encore plus particulièrement de 5% à 60% massique d'au moins un tensioactif lipophile choisi parmi les esters de sorbitan.

La composition selon l'invention peut se présenter sous différentes formes solides à température ambiante (billes, sphères, écailles, paillettes, perles...). Elle est principalement destinée à la mise en forme d'actifs dans les secteurs de l'industrie pharmaceutique, animale ou humaine, thérapeutique et/ou prophylactique, les industries des compléments alimentaires et/ou de l'alimentation humaine et animale.

Au sens de la présente invention, on entend par excipient lipidique un excipient ayant une température de fusion inférieure ou égale à 120° C, préférentiellement inférieure ou égale à 100°C et solide à température ambiante (supérieure ou égale à 15°C et inférieure ou égale à 30°C), insoluble ou peu soluble dans l'eau.

La cire d'abeille mise en œuvre dans la composition selon l'invention est jaune ou blanche, est aussi désignée par le numéro E901 et est de point de fusion compris entre 60 et 67°C.

Concernant le tensioactif lipophile, on précisera ici que les termes lipophiles, et hydrophiles, sont des termes relatifs. Un paramètre empirique communément utilisé pour caractériser l'hydrophilie et la lipophilie relative des composés amphiphiles non ioniques est l'équilibre hydrophile-lipophile, soit la valeur dite de "HLB".

Ainsi, les tensioactifs ayant des valeurs de HLB plus faibles sont plus lipophiles et ont une plus grande solubilité dans les huiles, tandis que les tensioactifs avec des valeurs de HLB plus élevées sont plus hydrophiles et ont une plus grande solubilité dans les solutions aqueuses.

En utilisant les valeurs HLB à titre indicatif, les tensioactifs hydrophiles sont généralement considérés comme étant des composés ayant une valeur HLB supérieure ou égale à 10, ainsi que des composés anioniques, cationiques ou zwitterioniques pour lesquels l'échelle HLB n'est généralement pas applicable. De même, les tensioactifs lipophiles sont des composés ayant une valeur HLB inférieure à environ 10. Dans les deux cas, est mentionné le terme environ du fait d'une variabilité induite.

Selon le cas, la composition lipidique selon l'invention peut présenter une ou plusieurs des caractéristiques ci-dessous :
- l'excipient lipidique est choisi parmi les cires animales, les cires végétales, les cires minérales, les cires synthétiques ou les huiles végétales hydrogénées ;
- le tensioactif lipophile est un tensioactif lipophile de la famille des esters de sorbitan, plus particulièrement un élément du groupe constitué par le monolaurate de sorbitan, le monopalmitate de sorbitan, le monostéarate de sorbitan et le monooléate de sorbitan, et encore plus particulièrement le monopalmitate de sorbitan ou le monostéarate de sorbitan, et encore plus particulièrement le monostéarate de sorbitan.
- ladite composition comprend de 0 à 20% massique d'un ou plusieurs tensioactifs hydrophiles, plus particulièrement de 0% à 10% massique.
- le ou les tensioactifs hydrophiles sont choisis parmi la lécithine de soja, les esters de sorbitan polyéthoxylés, les alcools polyéthoxylés, les acides polyéthoxylés, les esters de polyglycérol, les éthers de glucose et les copolymères blocs d'oxydes d'éthylène et de propylène.
- le tensioactif lipophile est un ester de sorbitan et le tensioactif hydrophile est choisi parmi les esters de sorbitan polyéthoxylés.
- ladite composition comprend : 80% de cire d'abeille, 20% de stéarate de sorbitan
- ladite composition comprend : 94% de cire d'abeille, 1% de stéarate de sorbitan, et 5% d'oléate de sorbitan polyéthoxylé à 20 moles d'oxyde d'éthylène (ou également dénommé de polysorbate 80).
- ladite composition comprend : 75% de cire d'abeille, 20% de stéarate de sorbitan, et 5% d'oléate de sorbitan polyéthoxylé à 20 moles d'oxyde d'éthylène (ou également dénommé polysorbate 80).
- ladite composition comprend : 50% de cire d'abeille, 45% de stéarate de sorbitan, et 5% d'oléate de sorbitan polyéthoxylé à 20 moles d'oxyde d'éthylène (ou polysorbate 80).
- ladite composition comprend de 0 à 20% massique, et plus particulièrement de 0 à 10% massique d'au moins un adjuvant d'enrobage.
- le ou les adjuvants d'enrobage sont choisis parmi les diluants, les arômes, les appétants, les colorants, les antioxydants, les agents plastifiants, les anti-mousses et les désintégrants.

Concernant l'excipient lipidique :
- pour les cires animales on peut citer : le spermateci (blanc de baleine) qui présente un point de fusion compris entre 52 et 55°C, la lanoline qui présente un point de fusion entre 37 et 44°C, et la gomme laque qui présente un point de fusion entre 77 et 90°C ;
- pour les cires végétales on peut citer: la cire de carnauba qui présente un point de fusion entre 78 et 88°C, la cire de candelilla qui présente un point de fusion entre 67-79°C, et la cire de son de riz qui présente un point de fusion proche de 78°C ;
- pour les cires minérales on peut citer : la paraffine qui présente un point de fusion entre 50 et 71°C, et la microcrystalline wax qui présente un point de fusion entre 54 et 102°C ;
- pour les cires synthétiques on peut citer : les cires Fischer-Tropsch, les cires de polyéthylène (ou polypropylène), les cires de poly(oxyde d'éthylène) ou poly(oxyde de propylène)...
- pour les huiles végétales hydrogénées on peut citer: l'huile de palme qui présente un point de fusion entre 58 et 62°C) et la stéarine qui présente un point de fusion entre 61et 65°C.

Parmi les tensioactifs lipophiles, on peut citer les esters de sorbitan que l'on peut associer à la composition objet de la présente invention, on peut citer :
- le monolaurate de sorbitan commercialisé par la société SEPPIC sous le nom de marque Montane^{™}20, par la société Croda sous le nom de marque Span^{™}20 et par la société,
- le monopalmitate de sorbitan commercialisé par la société SEPPIC sous le nom de marque Montane^{™}40, par la société Croda sous le nom de marque Span^{™}40,
- le monostéarate de sorbitan commercialisé par la société SEPPIC sous le nom de marque Montane^{™}60, par la société Croda sous le nom de marque Span^{™}60,
- le monooléate de sorbitan commercialisé par la société SEPPIC sous le nom de marque Montane^{™}80, par la société Croda sous le nom de marque Span^{™}80,
- le trioléate de sorbitan commercialisé par la société SEPPIC sous le nom de marque Montane^{™}85, par la société Croda sous le nom de marque Span^{™}85,
- le sesquioléate de sorbitan commercialisé par la société SEPPIC sous le nom de marque Montane^{™}83, par la société Croda sous le nom de marque Crill^{™}43 et par la société,
- le tristéarate de sorbitan commercialisé par la société SEPPIC sous le nom de marque Montane^{™}65, par la société Croda sous le nom de marque Span^{™}65,

- le monoisostéarate de sorbitan commercialisé par la société SEPPIC sous le nom de marque Montane^{™}70, par la société Croda sous le nom de marque Crill^{™}6.

La composition selon l'invention comprend éventuellement de 0 à 20% d'un ou plusieurs tensioactifs hydrophiles choisis parmi la lécithine de soja, les esters de sorbitan éthoxylés, les alcools polyéthoxylés, les acides polyéthoxylés, les esters de polyglycérol, les éthers de glucose et les copolymères blocs d'oxydes d'éthylène et de propylène.

Parmi les esters de sorbitan éthoxylés que l'on peut associer à la composition objet de la présente invention, on peut citer :
- le monolaurate de sorbitan éthoxylé à 20 moles d'oxyde d'éthylène commercialisé par la société SEPPIC sous le nom de marque Montanox^{™}20, et par la société Croda sous le nom de marque Tween^{™}20,
- le monolaurate de sorbitan éthoxylé à 4 moles d'oxyde d'éthylène, commercialisé sous le nom de marque Tween^{™}21,
- le monolaurate de sorbitan éthoxylé à 6 moles d'oxyde d'éthylène, commercialisé sous le nom de marque Nikkol^{™} GL-I par la société Nikko,
- le monopalmitate de sorbitan éthoxylé à 20 moles d'oxyde d'éthylène commercialisé par la société SEPPIC sous le nom de marque Montanox^{™} 40, et par la société Croda sous le nom de marque Tween^{™}40,
- le monostéarate de sorbitan éthoxylé à 20 moles d'oxyde d'éthylène commercialisé par la société SEPPIC sous le nom de marque Montanox^{™} 60, et par la société Croda sous le nom de marque Tween^{™} 60,
- le tristéarate de sorbitan éthoxylé à 20 moles d'oxyde d'éthylène commercialisé par la société SEPPIC sous le nom de marque Montanox^{™} 65, et par la société Croda sous le nom de marque Tween^{™} 65,
- le monooléate de sorbitan éthoxylé à 20 moles d'oxyde d'éthylène commercialisé par la société SEPPIC sous le nom de marque Montanox^{™} 80, et par la société Croda sous le nom de marque Tween^{™} 80,
- le trioléate de sorbitan éthoxylé à 20 moles d'oxyde d'éthylène commercialisé par la société SEPPIC sous le nom de marque Montanox^{™} 85, et par la société Croda sous le nom de marque Tween^{™} 85,
- le monoisostéarate de sorbitan éthoxylé à 20 moles d'oxyde d'éthylène commercialisé par la société SEPPIC sous le nom de marque Montanox^{™} 70, et par la société Croda sous le nom de marque Tween^{™} 120,
- le monostéarate de sorbitan éthoxylé à 4 moles d'oxyde d'éthylène commercialisé par la société Croda sous le nom de marque Tween^{™} 61,
- le monooléate de sorbitan éthoxylé à 5 moles d'oxyde d'éthylène commercialisé par la société SEPPIC sous le nom de marque Montanox^{™} 81, et par la société Croda sous le nom de marque Tween^{™} 81.

Parmi les alcools gras éthoxylés que l'on peut associer à la composition objet de la présente invention comme tensioactifs hydrophiles, on peut citer l'alcool oléique éthoxylé à 2 moles d'oxyde d'éthylène, l'alcool oléique éthoxylé à 3 moles d'oxyde d'éthylène, l'alcool oléique éthoxylé à 5 moles d'oxyde d'éthylène, l'alcool oléique éthoxylé à 10 moles d'oxyde d'éthylène, l'alcool oléique éthoxylé à 20 moles d'oxyde d'éthylène, l'alcool laurique éthoxylé à 4 moles d'oxyde d'éthylène, l'alcool laurique éthoxylé à 7 moles d'oxyde d'éthylène, l'alcool laurique éthoxylé à 9 moles d'oxyde d'éthylène, l'alcool laurique éthoxylé à 23 moles d'oxyde d'éthylène, l'alcool cétylique éthoxylé à 2 moles d'oxyde d'éthylène, l'alcool cétylique éthoxylé à 10 moles d'oxyde d'éthylène, l'alcool cétylique éthoxylé à 20 moles d'oxyde d'éthylène, l'alcool stéarylique éthoxylé à 2 moles d'oxyde d'éthylène, l'alcool stéarylique éthoxylé à 10 moles d'oxyde d'éthylène, l'alcool stéarylique éthoxylé à 20 moles d'oxyde d'éthylène, l'alcool stéarylique éthoxylé à 100 moles d'oxyde d'éthylène.

Parmi les acides gras éthoxylés que l'on peut associer à la composition objet de la présente invention comme tensioactifs hydrophiles, on peut citer l'acide monolaurique éthoxylé entre 4 et 200 moles d'oxyde d''éthylène, et plus particulièrement l'acide monolaurique éthoxylé à 4 moles d'oxyde d'éthylène, l'acide monolaurique éthoxylé à 6 moles d'oxyde d'éthylène, l'acide monolaurique éthoxylé à 7 moles d'oxyde d'éthylène, l'acide monolaurique éthoxylé à 8 moles d'oxyde d'éthylène, l'acide monolaurique éthoxylé à 10 moles d'oxyde d'éthylène, l'acide monolaurique éthoxylé à 50 moles d'oxyde d'éthylène, l'acide monolaurique éthoxylé à 100 moles d'oxyde d'éthylène, l'acide monolaurique éthoxylé à 200 moles d'oxyde d'éthylène, l'acide monooléique éthoxylé entre 4 et 200 moles d'oxyde d"éthylène, et plus particulièrement l'acide monooléique éthoxylé à 1 mole d'oxyde d'éthylène, l'acide monooléique éthoxylé à 2 moles d'oxyde d'éthylène, l'acide monooléique éthoxylé à 4 moles d'oxyde d'éthylène, l'acide monooléique éthoxylé à 5 moles d'oxyde d'éthylène, l'acide monooléique éthoxylé à 6 moles d'oxyde d'éthylène, l'acide monooléique éthoxylé à 8 moles d'oxyde d'éthylène, l'acide monooléique éthoxylé à 9 moles d'oxyde d'éthylène, l'acide monooléique éthoxylé à 10 moles d'oxyde d'éthylène, l'acide monooléique éthoxylé à 50 moles d'oxyde d'éthylène, l'acide monooléique éthoxylé à 100 moles d'oxyde d'éthylène, l'acide monooléique éthoxylé à 200 moles d'oxyde d'éthylène, l'acide monostéarique éthoxylé entre 4 et 200 moles d'oxyde d"éthylène, et plus particulièrement l'acide monostéarique éthoxylé à 1 mole d'oxyde d'éthylène, l'acide monostéarique éthoxylé à 2 moles d'oxyde d'éthylène, l'acide monostéarique éthoxylé à 4 moles d'oxyde d'éthylène, l'acide monostéarique éthoxylé à 5 moles d'oxyde d'éthylène, l'acide monostéarique éthoxylé à 6 moles d'oxyde d'éthylène, l'acide monostéarique éthoxylé à 8 moles d'oxyde d'éthylène, l'acide monostéarique éthoxylé à 9 moles d'oxyde d'éthylène, l'acide monostéarique éthoxylé à 10 moles d'oxyde d'éthylène, l'acide monostéarique éthoxylé à 50 moles d'oxyde d'éthylène, l'acide monostéarique éthoxylé à 100 moles d'oxyde d'éthylène, l'acide monostéarique éthoxylé à 200 moles d'oxyde d'éthylène, l'acide monostéarique éthoxylé à 300 moles d'oxyde d'éthylène, l'acide monostéarique éthoxylé à 1000 moles d'oxyde d'éthylène.

Parmi les huiles de ricin hydrogénées et éthoxylées que l'on peut associer à la composition objet de la présente invention comme tensioactifs hydrophiles, on peut citer l'huile de ricin hydrogénée et éthoxylée à 5 moles d'oxyde d'éthylène, l'huile de ricin hydrogénée et éthoxylée à 7 moles d'oxyde d'éthylène, l'huile de ricin hydrogénée et éthoxylée à 10 moles d'oxyde d'éthylène, l'huile de ricin hydrogénée et éthoxylée à 20 moles d'oxyde d'éthylène, l'huile de ricin hydrogénée et éthoxylée à 25 moles d'oxyde d'éthylène, l'huile de ricin hydrogénée et éthoxylée à 30 moles d'oxyde d'éthylène, l'huile de ricin hydrogénée et éthoxylée à 40 moles d'oxyde d'éthylène, l'huile de ricin hydrogénée et éthoxylée à 45 moles d'oxyde d'éthylène, l'huile de ricin hydrogénée et éthoxylée à 50 moles d'oxyde d'éthylène, l'huile de ricin hydrogénée et éthoxylée à 60 moles d'oxyde d'éthylène, l'huile de ricin hydrogénée et éthoxylée à 80 moles d'oxyde d'éthylène, l'huile de ricin hydrogénée et éthoxylée à 100 moles d'oxyde d'éthylène.

La composition selon l'invention comprend éventuellement de 0 à 20% d'au moins un adjuvant d'enrobage choisis parmi les diluants, les agents plastifiants, les anti-mousses et les désintégrants.

Parmi les diluants que l'on peut associer à la composition objet de la présente invention on peut citer : le lactose, le sucrose, le mannitol, le sorbitol, le xylose, le xylitol, l'isomalt, le talc, les amidons natifs, la silice, le dioxyde de silice et le stéarate de magnésium.

Parmi les agents plastifiants que l'on peut associer à la composition objet de la présente invention on peut citer :la glycérine, les polypropylène glycols, les polyéthylènes glycols ou leurs dérivés de condensation avec un acide gras ou un alcool gras, l'acide stéarique et ses dérivés, les mono glycérides acétylés, les esters de l'acide citrique comme par exemple le triéthyl citrate, l'acétyl triéthyl citrate, l'acétyl tributyl citrate, la triacétine, le sorbitol, le dibutyl seccate.

Parmi les anti-mousses que l'on peut associer à la composition objet de la présente invention on peut citer les dérivés de silicone.

Parmi les désintégrants que l'on peut associer à la composition objet de la présente invention on peut citer : les dérivés de cellulose, les crospovidones, croscarmelloses de sodium, sodium starch glycolate

Notons que d'autres adjuvants tels que les parfums, les arômes, les appétants, les colorants, les pigments et les antioxydants peuvent être associés à la composition objet de la présente invention.

La présente invention a également pour objet une composition (C_{A}) à libération contrôlée comprenant :
- au moins une composition lipidique selon l'invention, et
- au moins une substance active pharmaceutique, prophylactique ou alimentaire.
Cette composition (C_{A}) selon l'invention sera de préférence destinée à être administrée oralement chez l'être humain ou animal.
Selon un autre mode préférentiel, la composition (C_{A}) selon l'invention sera de forme solide.

Parmi les principes actifs pharmaceutiques, on peut citer les anti-rhumatismaux et anti-inflammatoires non stéroïdiens (ketoprofène, ibuprofène, flurbiprofène, indométacine, phénylbutazone, allopurinol...), les analgésiques (paracétamol, phénacétine, aspirine...), les antitussifs (codéine, codéthyline, alimemazine...), les stérols (hydrocortisone, cortisone, progestérone, testostérone, triamcinolone, dexamethazone, betamethazone, paramethazone, fluocinolone, beclomethazone...), les barbituriques (barbital, allobarbital, phenobarbital, pentobarbital, amobarbital...), les antimicrobiens (pefloxacine, sparfloxacine, et dérivés de la classe des quinolones, tetracylines, synergistines, metronidazole...), les médicaments destinés au traitement des allergies, les anti-asthmatiques, les vitamines (vitamine A, vitamines B, vitamine C, vitamine E, vitamines du groupe D, vitamine K), les antispasmodiques et anti sécrétoires (omeprazole), les cardiovasculaires et les vasodilatateurs cérébraux (quinacainol, oxprenolol, propanolol, nicergotine...), les protecteurs cérébraux, les protecteurs hépatiques les agents thérapeutiques du tractus gastro intestinal, les vaccins, les antihypertenseurs et les cardioprotecteurs tels que les bétabloquants et les dérivés nitrés.

Parmi les agents nutritionnels, on peut citer des principes actifs habituellement mis en œuvre dans le domaine de la nutrition, comme des lipides bioactifs, les sels d'oligo-éléments hydrosolubles ou hydro-dispersibles, les vitamines hydrosolubles ou liposolubles, les prébiotiques, les probiotiques, les protéines et/ou les concentrats de protéines laitières, les enzymes végétales ou animales, les acides aminés, les peptides, les sucres, les exhausteurs de goût, les agents aromatisants, les ingrédients botaniques ( les extraits végétaux de gingembre, de curcumin, de millepertuis, de valériane, les extraits de myrtille, les extraits de grenades, les extraits de chorella vulgaris, les extraits d'artichauts, les extraits d'hibiscus).

Parmi les lipides bioactifs, on peut citer les phytostérols, comme ceux extraits des huiles végétales, et plus particulièrement les extraits de l'huile d'argousier, de l'huile de maïs, de l'huile de soja ; les complexes de phytostérols, isolés des huiles végétales, comme par exemple la cholestatine, composée de campestérol, de stigmastérol et de brassicastérol ; les phytostanols ; les caroténoïdes, qui appartiennent à la famille des terpénoïdes, extraits des algues, des plantes vertes, des champignons, des bactéries ; les acides gras polyinsaturés du groupe oméga-3, comme par exemple l'acide alpha-linolénique, l'acide eicosapentaénoïque, l'acide docosahexanoïque ; les acides gras polyinsaturés du groupe oméga-6, comme par exemple l'acide linoléique, l'acide γ-linolénique, l'acide
Parmi les sels d'oligo-éléments hydrosolubles ou hydro-dispersibles utilisés dans des formes solides ingérables enrobées avec la composition d'enrobage objet de la présente invention, on peut citer le carbonate ferreux, le chlorure ferreux tétrahydraté, le chlorure ferrique hexahydraté, le citrate ferreux hexahydraté, le fumarate ferreux, le lactate ferreux tétrahydraté, le sulfate ferreux monohydraté, le sulfate ferreux heptahydraté, le chélate ferreux d'acides aminés hydratés, le chélate de fer de glycine ; l'iodate de calcium hexahydraté, l'iodate de calcium anhydre ; l'iodure de sodium, l'iodure de potassium ; l'acétate de cobalt tétrahydraté, le carbonate basique de cobalt monohydraté, le carbonate de cobalt hexahydraté, le sulfate de cobalt heptahydraté, le sulfate de cobalt monohydraté, le nitrate de cobalt hexahydraté ; l'acétate cuivrique monohydraté, le carbonate basique de cuivre monohydraté, le chlorure cuivrique dihydraté, le méthionate de cuivre, le sulfate cuivrique pentahydraté, le chélate cuivreux d'acides aminés hydratés, le chélate cuivreux de glycine hydraté, le chélate de cuivre de Phydroxy-analogue de méthionine ; le carbonate manganeux, le chlorure manganeux tétrahydraté, le phosphate acide de manganèse trihydraté, le sulfate manganeux tétrahydraté, le sulfate manganeux monohydraté, le chélate de manganèse d'acides amines hydraté, le chélate de manganèse de glycine hydraté, le chélate de manganèse de l'hydroxy-analogue de méthionine ; le molybdate d'ammonium, le molybdate de sodium, le sélénite de sodium, le sélénate de sodium ; la forme organique du sélénium produite par saccharomyces cerevisiae, la sélénomethionine (levure séléniée inactivée), et la séléno méthionine produite par Saccharomyces cerevisiae (levure séléniée inactivée).

Parmi les sels minéraux, on peut citer les sels de cations métalliques, comme par exemple les cations du sodium, du potassium, du calcium, du magnésium, du zinc, du manganèse, du fer, du cuivre, du cobalt, de l'argent, du baryum, du zirconium et du strontium, et d'anions organiques, comme par exemple un anion organique comestible, possédant au moins une fonction acide carboxylique sous forme carboxylate, choisi parmi les éléments du groupe constitué par les anions issus des acides glycolique, citrique, tartrique, salicylique, lactique, mandélique, ascorbique, pyruvique, fumarique, glycérophosphorique, rétinoïque, benzoïque, kojique, malique, gluconique, galacturonique, propionique, heptanoïque, 4-amino benzoïque, cinnamique, benzalmalonique, aspartique et glutamique.

Parmi les sels minéraux, on peut citer plus particulièrement le gluconate de zinc, le gluconate de calcium, le gluconate de manganèse, le gluconate de cuivre, l'aspartate de magnésium, l'aspartate de calcium, le glycérophosphate de calcium, calcium, le glycérophosphate de magnésium.

Parmi les vitamines hydrosolubles ou liposolubles, on peut citer la vitamine A, plus particulièrement sous sa forme de rétinol, d'acétate de rétinyle, de palmitate de rétinyle ou de bêta15 carotène ; la vitamine D2, plus particulièrement sous sa forme dergocalciférol, ou de -hydroxy calciférol, la vitamine D3, plus particulièrement sous sa forme de cholécalciférol, la vitamine K, plus particulièrement sous sa forme de phylloquinone (phytoménadione) ou de Ménaquinone, la vitamine B1, plus particulièrement sous sa forme de chlorhydrate de thiamine, de mononitrate de thiamine, de chlorure de thiamine monophosphate, ou de chlorure de thiamine pyrophosphate, la vitamine B2, plus particulièrement sous sa forme de riboflavine, de riboflavine 5'-phosphate de sodium, la vitamine B6, plus particulièrement sous sa forme de chlorhydrate de pyridoxine, de pyridoxine 5'- phosphate, ou de pyridoxal 5'-phosphate, la vitamine B12, plus particulièrement sous sa forme de cyanocobalamine, d'hydroxocobalamine, de 5'- déoxyadénosylcobalamine, ou de méthylcobalamine, la vitamine C, plus particulièrement sous sa forme d'acide L- ascorbique, de L-ascorbate de sodium, de L-ascorbate de calcium, de L-ascorbate de potassium, de sels de calcium de l'acide palmityl-6-L-ascorbique, de sodium ascorbylmonophosphate, l'acide pantothénique, plus particulièrement sous sa forme de D-pantothénate de calcium, de D-pantothénate de sodium, de D-expanthénol, ou de Pantéthine, la vitamine PP, plus particulièrement sous sa forme d'acide nicotinique, de niacine, de nicotinamide, ou d'hexanicotinate d'inositol (hexaniacinate d'inositol), la vitamine B9, plus particulièrement sous sa forme d'acide folique, les folates, plus particulièrement sous leur forme d'acide ptéroylmonoglutamique, de L-méthylfolate de calcium, d'acide (6S)-5- 5 méthyltétrahydrofolique sous forme de sel de glucosamine, la vitamine H2, B7 ou BW, plus particulièrement sous sa forme de biotine, la choline, plus particulièrement sous sa forme de chlorure de choline, de choline dihydrogène citrate, de bitartrate de choline, l'inositol, la carnitine, plus particulièrement sous sa forme de L-carnitine, L-carnitine-L-tartrate, la taurine.

Parmi les pré-biotiques, on peut citer l'inuline, les transgalactooligosaccharides, les fructanes et les mannooligosaccharides.

Parmi les pro-biotiques, on peut citer différentes souches de Saccharomyces cerevisiae, de Bacillus cereus var toyoi, de Bacillus subtilis seul ou en combinaison avec le Bacillus licheniformis, ou encore des souches d'Enteroccocus faecium, les bactéries lactiques et plus particulièrement les lactobacilles, les bifidobactéries et des streptocoques. Ces souches de microorganismes sont généralement associées à un support solide, par exemple le carbonate de calcium, le dextrose ou le sorbitol.

Parmi les protéines et/ou les concentrats de protéines, on peut citer les protéines laitières issues du cracking du lait, tels que le colostrum sous forme de poudre lyophilisée ou atomisée, le lactoserum sous forme de poudre, de fractions purifiées ou enrichies en IgG, en lactoferrine, en lactoperoxydase. Parmi les enzymes végétales ou animales, on peut citer la promutase, la superoxyde dismutase (SOD), la 3-phytase, la 6-phytase, les endo-l,4-betaglucanases, les endo-l,4- betaxylanases, ou encore d'autres enzymes améliorant ou favorisant la digestion.

Parmi les peptides, on peut citer les peptides de l'avocat, les peptides de lupin, les peptides de quinoa, les peptides de maca, les peptides de soja fermente ou non, les peptides de riz, les peptides présents dans l'extrait de graines d'Acacia macrostachya, les peptides présents dans les extraits de graines de passiflore.

Parmi les acides aminés, on peut citer l'alanine, l'arginine, l'asparagine, l'acide aspartique, la cystéine, l''acide glutamique, la glutamine, la glycine, l'histidine, l'isoleucine, la leucine, la lysine, la méthionine, la phénylalanine, la proline, l'hydroxy-proline, la pyrrolysine, la sélméno-cystéine, la sérine, la thréonine, le tryptophane, la tyrosine, la valine, la sarcosine, l'ornithine.

Parmi les sucres, on peut citer les polysaccharides solubles dans l'eau, les sucres de poids moléculaire inférieur, tels que les oligosaccharides, mono- ou disaccharides, comme par exemple le glucose, le lactose, le dextrose.
Parmi les exhausteurs de goût, on peut citer les glutamates, comme par exemple l'acide glutamique, le glutamate monosodique, le glutamate monopotassique, le diglutamate de calcium, le glutamate d'ammonium, le diglutamate de magnésium ; les guanylates, comme par exemple l'acide guanylique (guanosine monophosphate), le guanylate disodique, le Guanylate dipotassique, le Guanylate de calcium, les inosinates, comme par exemple l'acide inosinique, l'inosinate disodique, l'inosinate dipotassique, l'inosinate de calcium, ou encore des édulcorants intenses tels que les extraits de Stevia, les Rébiaudosides.

La présente invention a également pour objet l'utilisation d'une composition lipidique selon l'invention pour l'encapsulation d'une substance active pharmaceutique, thérapeutique, prophylactique, ou alimentaire pour l'être humain ou animal dans une formulation galénique.

Par encapsulation, on entend au sens de la présente invention une opération permettant de mettre en commun une composition lipidique et un ou des principes actifs. Le ou les actifs pouvant se retrouver à l'intérieur d'une coque formée par cette composition lipidique "refroidie" ou dispersé(s) au sein de celle-ci.

Enfin la présente invention a pour dernier objet un procédé de fabrication d'une formulation galénique comprenant une composition (CA) selon l'invention, comprenant au moins :
a) une étape de préparation de la composition lipidique selon l'invention,
b) une étape de mélange et d'encapsulation d'une substance active pharmaceutique, prophylactique ou alimentaire avec la composition lipidique préparée à l'étape a), de manière obtenir une composition (C_{A}) selon l'invention, et
c) une étape de mise en forme galénique de la composition (C_{A}) préparée à l'étape b), impliquant un stress mécanique.

Par « stress mécanique » on entend une tension ou une pression qui agit sur un matériau et qui peut en changer la forme ou les propriétés. Dans le cadre de l'invention, il s'agit d'une contrainte exercée sur la composition CA lors de son utilisation pour préparer la forme galénique finale (étape c).
Toujours dans la cadre de l'invention notons que le profil de libération de la substance active dans la forme galénique finale est similaire ou peu différent du profil de libération de la substance active dans la composition CA. En effet, c'est grâce à la composition lipidique selon l'invention qu'on observe une résistance au stress mécanique et donc pas ou peu de modification du profil de libération.
Parmi les procédés d'encapsulation utilisés dans l'étape b), on peut citer le Prilling, le refroidissement par pulvérisation au froid ou Spray chilling ou Spray cooling, de congélation par pulvérisation ou Spray congealing, de revêtement en milieu fondu ou Hot Melt Coating, d'extrusion en milieu fondu ou Hot Melt Extrusion,, de granulation en milieu fondu ou Melt granulation, de pastillage ou Pelletisation, de sphéronisation, de granulation thermique ou Thermogranulation... (mettre la traduction en français si possible).

Par "Prilling", on désigne un procédé d'enrobage par solubilisation ou dispersion du ou des principes actifs dans une composition lipidique fondue, puis pulvérisation dans un air ambiant ou refroidi, ou un liquide refroidi

Les procédés "Spray chilling", "spray cooling" et "spray congealing" sont des procédés particuliers du procédé de prilling.

Par "Hot Melt coating", on désigne un procédé d'enrobage par pulvérisation d'une composition lipidique fondue sur une particule solide, constituée de l'actif ou d'un mélange d'actifs.

Selon le cas le procédé de fabrication selon l'invention peut présenter une ou plusieurs des caractéristiques suivantes :
- l'étape b) de mélange et d'encapsulation comprend une première sous-étape de chauffage de la composition lipidique préparée à l'étape a) à une température supérieure de 10 à 15°C de la plus haute température de fusion des différents ingrédients de ladite composition lipidique de manière à faire fondre ladite composition lipidique, une deuxième sous-étape de mélange de la composition lipidique fondue avec la substance active sous forme dispersée ou fondue, et une troisième sous-étape de pulvérisation de la composition obtenue à la deuxième sous-étape dans un air ambiant ou refroidi ou dans un liquide refroidi afin d'obtenir des particules solidifiées de composition (C_{A}) ; Par « air ambiant » on entend de l'air à une température ambiante généralement aux alentours de 25°C; par « air refroidi » ou « liquide refroidi » on entend de l'air ou un liquide à une température inférieure à la température ambiante, autrement dit inférieure à 25°C ;
- l'étape de mise en forme galénique impliquant un stress mécanique est choisie parmi les procédés de mise en forme posologique solide ("solid oral dosage forms"), et par exemple la compression, la mise en gélules ("capsule filling" / "molding"), le compactage ("compaction", "roller compaction"), la mise en emballage, la mise en sachets, la mise en sticks ("packaging", "stick" and "sachet filling"), l'extrusion, la granulation, la pelletisation ; ces techniques pouvant conduire à la fabrication des formes suivantes (non exhaustif) : poudre orale (oral powder), billes (beads), gélules (capsules), granules, granulés, perles, pellets, sphères (spherules), comprimés (tablets), sachets, sticks, gommes à mâcher (chew, gums), comprimés à croquer (chewable tablets), chewing gums.

La composition lipidique selon l'invention permet une mise en forme d'une substance active par différentes technologies d'enrobages et induisant de manière inattendue une stabilité du profil de libération de la substance active ainsi enrobée, par rapport au profil de libération de la substance active dont la forme galénique n'a pas été obtenue par un procédé comprenant une étape de stress mécanique.

Autrement dit, l'objet de la présente invention permet de maintenir le profil de libération de la substance active initialement recherché et ce quel que soit le processus de mise en forme galénique aval réalisé, quel que soit le stress mécanique que l'on applique pour mettre en forme produit fini l'actif (mélange, compaction, mise en gélule, mise en stick, mise en comprimé...).

Au sens de la présente invention, on entend par « Mise en forme selon des technologies d'enrobage d'actifs » toute technologie primaire ou tout procédé permettant de conférer à la composition comprenant ledit actif une forme physique, de préférence une forme solide à température ambiante.
Les exemples suivants illustrent l'invention. Les revendications définissent la portée de l'invention.

### Exemples

La qualification des compositions d'encapsulation telles que décrites ci-dessus induisant des propriétés de résistances aux stress mécaniques des étapes de galéniques avals, est établie par la mise en place d'analyses telles que décrites ci-dessous.

Un actif est choisi comme actif modèle principal. Notons que par « actif » on entend une substance active. Il s'agit de la caféine, de solubilité moyenne à température ambiante (25°C) dans l'eau de 20-25g/l.
Formule moléculaire : C8H10N4O2 ; poids moléculaire : 194.194 g/mol.

Cet actif est encapsulé avec les compositions objets de la présente invention à un taux de 20% à 30% par technologie de prilling à disque tournant.

### Technologie de prilling par disque tournant : procédé de préparation de la composition M1

Pour chaque essai, une dispersion cireuse liquide est préparée. Cette dispersion cireuse liquide est constituée :
- De la composition objet de la présente invention, sous forme fondue ;
- De l'actif modèle, sous forme solide dispersé. Dans le cas général, on rappelle qu'un actif peut être présent sous forme dispersée ou liquide selon la valeur de sa température de fusion

Pour cela, une 'pré-dispersion', résultat de la fonte et/ou mélange à l'état liquide des différents éléments de la composition objet de la présente est préalablement réalisée : préparation mélange M0.

La température à laquelle est maintenue cette pré-dispersion puis la dispersion reconstituée doit être réglée de façon à être supérieure de 10 à 15°C de la plus haute température de fusion des différents ingrédients de la composition. Avant mise en route du process de fabrication, l'actif est ajouté, dispersé dans ce cas, sous agitation mécanique en maintenant la température préalablement établie.
Le processus de prilling par disque tournant est alors réalisé. La dispersion est convoyée au moyen de tuyaux calorifugés jusqu'à une buse de pulvérisation se situant dans un espace/tour, menant, après sprayage, à la création de fines gouttelettes. Ces fines gouttelettes sont alors solidifiées dans un courant d'air ambiant à froid, amenant à la formation de petites billes sphériques caractérisées en ce que 50% en volume de ces dites billes sont de diamètre compris entre 300 et 500µm. On notera que la mesure du profil granulométrique des billes est effectuée grâce à un granulomètre laser Mastersizer 3000 de MALVERN, utilisée en voie sèche, à une pression de 1bar.
Les microbilles ainsi réalisées peuvent être additionnées optionnellement de dioxyde de silicium ou autre agent d'écoulement/anti-collage avant d'en faciliter la manipulation postérieure.

De même, un tamisage sur tamis 500µm peut être réalisé afin d'éliminer tout résidu/agglomérat non désiré dont le diamètre de particules est supérieur ou égal à 500 µm.

Les microbilles ainsi obtenues sont ensuite incorporées dans un mélange d'excipients de grades compatibles avec un processus de mise en forme galénique de type comprimés. La forme comprimée est choisie comme modèle de processus de forme galénique finale car elle représente l'un des cas les plus extrêmes de stress mécanique induit.

La mise en forme comprimé est réalisée sur presse alternative mono-poinçon de type DOTT BONAPACE ou rotative 8 poinçons de type RIVA PICCOLA, instrumentées, avec application d'une force de compression pouvant varier entre 5 et 20 kN ; des comprimés de 500mg, de résistances à la rupture comprises entre 80 et 120 N, sont ainsi fabriqués.

### Mise en évidence de l'effet technique :

Le profil de libération de l'actif, enrobé dans la composition objet de la présente invention puis incorporé dans un format comprimé, est établi par la biais d'un testeur de dissolution ERWEKA, suivant les recommandations de la Pharmacopée Européenne, version 7.3, paragraphe 2.9.3.

Le milieu de dissolution choisi principalement est un tampon phosphate pH 7.2 maintenu à une température de 37°C. Des prélèvements sont effectués périodiquement, jusqu'à 6H. Les prélèvements sont ensuite analysés par dosage HPLC, phase inverse, avec détection UV, afin de déterminer la quantité d'actif présente dans chaque prélèvement et ainsi établir un profil de dissolution par échantillon à évaluer.

Les profils de dissolution, composition à composition ou comparaison avant/après application d'un stress mécanique, sont comparés afin de déterminer l'écart oui/non de deux profils entre eux ou d'un profil par rapport à un profil témoin.

Deux profils sont ainsi jugés similaires ou peu différents, et donc la composition est jugée comme améliorant la résistance aux stress mécanique, lorsque :
- L'écart temps à temps, dont le calcul est le suivant : $abs \left[%libéré\left(Txi\right)cps-%libéré\left(Txi\right)μbilles\right] \leq y %$ où :
   %libéré(Txi)cps = % d'actif libéré au point de prélèvement Txi, lorsque la formulation a subi un procédé de mise en forme en comprimés.
   %libéré(Txi)µbilles = % d'actif libéré au point de prélèvement Txi, lorsque la formulation n'a pas subi de stress mécanique
   et est en valeur y inférieur ou égal à 30+/-2% jusqu'à 2 heures de temps de dissolution et inférieur ou égal à 20+/-2% sur le temps restant d'évaluation.
   On pourra juger le profil similaire lorsque l'écart tout au long du test de dissolution est inférieur ou égal à 15%.
- Le taux de variation entre les valeurs avant et après application d'un stress mécanique est inférieur ou égal à 30% à partir de 3 heures de temps de dissolution. Le calcul est le suivant : $\frac{% lib é r é \left(Txi\right) cps - % lib é r é \left(Txi\right) μ billes}{%lib é r é \left(Txi\right) μ billes} \leq 30 % à partir de 180 min$ où :
   Txi >= 180minutes
   %libéré(Txi)cps = % d'actif libéré au point de prélèvement Txi, lorsque la formulation a subi un procédé de mise en forme en comprimés (impliquant une étape de compression).
   %libéré(Txi)µbilles = % d'actif libéré au point de prélèvement Txi, lorsque la formulation n'a pas subi de stress mécanique

### • Exemple 1 :

Dans cet exemple, des compositions lipidiques contenant en composant A une même cire d'abeille, une même quantité de Polysorbate 80, et une même proportion de différents tensioactifs lipophiles comme composant B. La constitution des compositions lipidiques préparée est consignée dans le tableau 1 ci-dessous. Ces compositions lipidiques sont mise en œuvre pour l'enrobage selon le procédé de prilling tel que décrit ci-dessus et avec la caféine comme ingrédient actif. L'objet de cet exemple est de montrer la validité de l'association de la cire d'abeille avec certains tensioactifs lipophiles, pour l'enrobage par le procédé de prilling de l'actif caféine, en observant le maintien ou non d'un profil de libération au cours du temps, de façon à éventuellement ne pas considérer comme pertinentes certaines associations de cire d'abeille et de tensioactifs lipophiles, avant de leur faire subir une étape de compression.

**Tableau 1**

| **Identification de la composition** | **CL 1** | **CL 2' (comparatif)** | **CL 3'** |
|---|---|---|---|
| Cire d'abeille | 80,0 % | 80,0 % | 80,0 % |
| Stéarate de sorbitan (Montane 60 SEPPIC) | 19,6 % | | |
| Glycerol distearate (EP name) | | 19,6 % | |
| Glycerol Monostearate (EP name) | | | 19,6 % |
| Polysorbate 80 (Montanox 80 SEPPIC) | 0,4 % | 0,4 % | 0,4 % |
| | **100%** | **100%** | **100%** |

L'actif est dispersé à 20% dans la composition. Le procédé de prilling est mise en oeuvre. Nous obtenons des microbilles de diamètre médian compris entre 350 et 400µm.

Ces microbilles sont mises en stabilités à température ambiante, à l'abri de la lumière, pour une durée minimale de 28 mois.

Les profils de dissolution des microbilles, après processus de fabrication ou après une durée de vie minimale de 28 mois, sont étudiés. Différents temps de prélèvement sont réalisés ; la comparaison de profil sera réalisée au point 120 minutes.

Le tableau 2 ci-dessous donne les valeurs de pourcentages de caféine libérée au point de prélèvement de 120 minutes :

**Tableau 2**

| **Identification de la composition // Temps de prélèvement** | **CL1'- A T0 de l'obtention des microbilles** | **CL 1' - A minimun T28mois de l'obtention des microbilles** | **CL2'- A T0 de l'obtention des microbilles** | **CL 2' - A minimun T28mois de l'obtention des microbilles** | **CL3'- A T0 de l'obtention des microbilles** | **CL 3' - A minimun T28mois de l'obtention des microbilles** |
|---|---|---|---|---|---|---|
| 120minutes | 71% | 75% | 73% | 64% | 100% | 75% |

Le tableau 3 ci-dessous mentionne les valeurs des écarts temps à temps* au point de prélèvement 120 minutes :

**Tableau 3**

| **Identification de la composition // Temps de prélèvement** | **CL1' - Ecart temps à temps** | **CL2' - Ecart temps à temps** | **CL3' - Ecart temps à temps** |
|---|---|---|---|
| 120 minutes | 4% | 9% | 25% |

| | | | |
|---|---|---|---|
| *(*) : il faut noter ici une définition spécifique de l'écart temps à temps, dans le cas d'une comparaison de profil de libération à T0 et après une période de mise en stabilité. Ainsi, l'écart temps à temps est ici redéfini comme étant issu du calcul suivant :*$abs \left[% libéré\left(120x′i\right)μbilles-% libéré\left(120xi\right)μbilles\right] y %$*où :* *% libéré (120x'i) µbilles = % d'actif libéré au point de prélèvement 120 minutes, lorsque la formulation a été stockée sur une durée définie à température ambiante.* *% libéré (120xi) µbilles = % d'actif libéré au point de prélèvement 120 minutes, lorsque la formulation n'a pas été stockée, soit valeur à T0 de production de la microbille.* | | | |

*Deux profils sont jugés de même ordre lorsque l'écart temps à temps est en valeur y inférieur ou égal à 20+*/*-2% au point de prélèvement considéré.*

*On pourra juger le profil similaire lorsque l'écart temps à temps est inférieur ou égal à 15%.*

Pour cet exemple, on peut observer que les compositions CL1' et CL2' permettent un maintien du profil de libération de l'actif caféine contenu dans les microbilles issues du processus de prilling, dans le temps. La composition CL3', contenant du Glycerol Monostearate en tant que composant B, ne peut d'ores et déjà pas être retenue.

### • Exemple 2 :

Dans cet exemple, des compositions lipidiques contenant en composant A des cires et huiles de différentes provenances qui sont mises en oeuvre pour l'enrobage par procédé de prilling de l'actif caféine tel que décrit ci-dessus. L'objet de cet exemple est de démontrer la spécificité de la cire d'abeille, associés aux autres composants, à permettre le maintien d'un profil de libération similaire/peu différent ou non de l'actif enrobé post process de mise en forme galénique/suite à stress mécanique.

Les compositions suivantes sont ainsi mises en parallèle (tableau 4) :

**Tableau 4**

| **Identification de la composition** | **CL 1** | **CL 2** | **CL 3** | **CL 4** |
|---|---|---|---|---|
| Cire d'abeille | 80,0 % | | | |
| Huile de palme | | | | 80,0 % |
| Cire de candelilla | | 80,0 % | | |
| Cire de carnauba | | | 80,0 % | |
| Stéarate de sorbitan | 19,6 % | 19,6 % | 19,6 % | 19,6 % |
| (Montane 60 SEPPIC) | | | | |
| Polysorbate 80 (Montanox 80 SEPPIC) | 0,4 % | 0,4 % | 0,4 % | 0,4 % |
| | **100%** | **100%** | **100%** | **100%** |

L'actif est dispersé à 20% dans la composition. Le procédé de prilling est mis en oeuvre. Nous obtenons des microbilles de diamètre médian compris entre 350 et 400µm.

Ces microbilles sont ensuite introduites dans un mélange pour comprimés. Des comprimés de 500mg, de diamètre 11 mm, sont ainsi produits, selon la formulation suivante : pour 40% massique de microbilles, on ajoute 27% massique de cellulose microcrystalline, 29% massique d'hydrogénophosphate de calcium dihydraté, 3% massique de crospovidone et 1% massique de stéarate de magnésium.

Les profils de dissolution des microbilles avant et après processus de compression sont étudiés. Les temps de prélèvement sont les suivants : 60, 120, 180, 240, 300 et 360 minutes.

Le tableau 5 ci-dessous donne les valeurs de pourcentages de caféine libérée en fonction du temps :

**Tableau 5**

| **Identification de la composition** // **Temps de prélèvement** | **CL1 - Avant processus de mise en forme galénique** | **CL1 - après mise en forme comprimés** | **CL2 - Avant processus de mise en forme galénique** | **CL2 - après mise en forme comprimés** | **CL3 - Avant processus de mise en forme galénique** | **CL3 - après mise en forme comprimés** | **CL4 - Avant processus de mise en forme galénique** | **CL 4 - après mise en forme comprimés** |
|---|---|---|---|---|---|---|---|---|
| 60minutes | 40% | 49% | 3% | 24% | 6% | 26% | 9% | 23% |
| 120minutes | 71% | 73% | 5% | 29% | 8% | 32% | 10% | 28% |
| 180minutes | 87% | 86% | 6% | 33% | 9% | 36% | 10% | 30% |
| 240minutes | > 90% | > 90% | 7% | 36% | 11% | 39% | 11% | 32% |
| 300minutes | > 90% | > 90% | 7% | 38% | 12% | 42% | 11% | 33% |
| 360minutes | > 90% | > 90% | 8% | 40% | 12% | 44% | 11% | 34% |

Le tableau 6 ci-dessous mentionne les valeurs des écarts temps à temps et les taux de variation à 180 minutes et au-delà :

**Tableau 6**

| **Identification de la composition // Temps de prélèvement** | **CL1 - Ecart temps à temps** | **CL 1 - Taux de variation** | **CL2 - Ecart temps à temps** | **CL 2 - Taux de variation** | **CL3 - Ecart temps à temps** | **CL 3 - Taux de variation** | **CL3 - Ecart temps à temps** | **CL3 - Taux de variation** |
|---|---|---|---|---|---|---|---|---|
| 60minutes | 9% | | 21% | | 20% | | 14% | |
| 120minutes | 2% | | 25% | | 24% | | 18% | |
| 180minutes | 0 | 0% | 27% | > 100% | 27% | > 100% | 20% | > 100% |
| 240minutes | N/A | N/A | 29% | > 100% | 29% | > 100% | 21% | > 100% |
| 300minutes | N/A | N/A | 31% | > 100% | 30% | > 100% | 22% | > 100% |
| 360minutes | N/A | N/A | 32% | > 100% | 32% | > 100% | 22% | > 100% |

Pour cet exemple, on peut voir que seule la composition CL1 permet une amélioration de la résistance à un stress mécanique, stress illustré ici par un processus de mise en forme galénique de type comprimés.

### • Exemple 3 :

Dans cet exemple, des compositions lipidiques contenant comme composant A un ratio de cire d'abeille et de cire de candelilla qui sont mises en oeuvre pour l'enrobage par procédé de prilling de l'actif caféine. L'objet de cet exemple est de démontrer la possibilité de mélange de la cire d'abeille avec un autre composé lipidique jusqu'à un certain ratio, associés aux autres composants, tout en permettant le maintien d'un profil de libération similaire/peu différent ou non de l'actif enrobé post process de mise en forme galénique/suite à stress mécanique.

Les compositions suivantes sont ainsi mises en parallèle :

**Tableau 7**

| **Identification de la composition** | **CL 1** | **CL 5** | **CL 6** (comparatif) |
|---|---|---|---|
| Cire d'abeille | 80,0% | 76,0% | 68,0% |
| Cire de candelilla | | 4,0% | 12,0% |
| Stéarate de sorbitan (Montane 60 SEPPIC) | 19,6 % | 19,6 % | 19,6 % |
| Polysorbate 80 (Montanox 80 SEPPIC) | 0,4 % | 0,4 % | 0,4 % |
| | **100%** | **100%** | **100%** |

L'actif est dispersé à 20% dans la composition. Le procédé de prilling est mise en oeuvre. Nous obtenons des microbilles de diamètre médian compris entre 350 et 400µm.

Ces microbilles sont ensuite introduites dans un mélange pour comprimés préparés selon le mode opératoire décrit dans l'exemple 2. Des comprimés de 500mg, de diamètre 11 mm, sont ainsi produits.

Les profils de dissolution des microbilles avant et après processus de compression sont étudiés. Les temps de prélèvement sont les suivants : 60, 120, 180, 240, 300 et 360 minutes.

Le tableau ci-dessous donne les valeurs de pourcentages de caféine libérée en fonction du temps :

**Tableau 8**

| **Identification de la composition** // **Temps de prélèvement** | **CL1 - Avant processus de mise en forme galénique** | **CL 1 - après mise en forme comprimés** | **CL5- Avant processus de mise en forme galénique** | **CL 5 - après mise en forme comprimés** | **CL6 - Avant processu s de mise en forme galénique** | **CL6 - après mise en forme comprimés** |
|---|---|---|---|---|---|---|
| 60minutes | 40% | 49% | 27% | 59% | 10% | 48% |
| 120minutes | 71% | 73% | 52% | 79% | 22% | 67% |
| 180minutes | 87% | 86% | 71% | 89% | 35% | 77% |
| 240minutes | > 90% | > 90% | 83% | > 90% | 46% | 84% |
| 300minutes | > 90% | > 90% | 90% | > 90% | 56% | 87% |
| 360minutes | > 90% | > 90% | > 90% | > 90% | 64% | 90% |

Le tableau ci-dessous mentionne les valeurs des écarts temps à temps et les taux de variation à 180minutes et au-delà :

**Tableau 9**

| **Identification de la composition // Temps de prélèvement** | **CL1 - Ecart temps à temps** | **CL 1 - Taux de variation** | **CL5 - Ecart temps à temps** | **CL 5 - Taux de variation** | **CL6 - Ecart temps à temps** | **CL6 - Taux de variation** |
|---|---|---|---|---|---|---|
| 60minutes | 9% | | 31% | | 38% | |
| 120minutes | 2% | | 26% | | 45% | |
| 180minutes | 0% | 0% | 19% | 26% | 43% | > 100% |
| 240minutes | NA | N/A | N/A | N/A | 37% | 80% |
| 300minutes | N/A | N/A | N/A | N/A | 31% | 56% |
| 360minutes | N/A | N/A | N/A | N/A | 25% | 39% |

Sur cet exemple, les compositions CL1 et CL5 permettent une amélioration de la résistance à un stress mécanique, avec un profil similaire pour la composition CL1, peu différent pour la composition CL5.

### • Exemple 4 :

Dans cet exemple, des compositions lipidiques présentant un composant A (cire d'abeille) et un composant B (un tensioactif hydrophobe de type ester de sorbitan) qui sont mises en oeuvre pour l'enrobage par procédé de prilling de l'actif caféine.

L'objet de cet exemple est de démontrer la possibilité de mélange du composant A et du composant B, pour un certain ratio, quant au maintien d'un profil de libération similaire/peu différent ou non de l'actif enrobé post process de mise en forme galénique/suite à stress mécanique.

Les compositions suivantes sont ainsi mises en parallèle :

**Tableau 10**

| **Identification de la composition** | **CL7** | **CL8** | **CL9** |
|---|---|---|---|
| Cire d'abeille | 100,0% | 80,0% | 60,0% |
| Stéarate de sorbitan (Montane 60 SEPPIC) | | 20,0% | 40,0% |
| | **100%** | **100%** | **100%** |

L'actif est dispersé à 20% dans la composition. Le procédé de prilling est mis en oeuvre. Nous obtenons des microbilles de diamètre médian compris entre 350 et 400µm.

Ces microbilles sont ensuite introduites dans un mélange pour comprimés préparés selon le mode opératoire décrit dans l'exemple 2. Des comprimés de 500mg, de diamètre 11 mm, sont ainsi produits.

Les profils de dissolution des microbilles avant et après processus de compression sont étudiés. Les temps de prélèvement sont les suivants : 30, 60, 120, 180, 240, 300 et 360 minutes.

Le tableau 11 ci-dessous donne les valeurs de pourcentages de caféine libérée en fonction du temps :

**Tableau 11**

| **Identification de** la **composition // Temps de prélèvement** | **CL7 - Avant processus de mise en forme galénique** | **CL7 - après mise en forme comprimés** | **CL8 - Avant processus de mise en forme galénique** | **CL8 - après mise en forme comprimés** | **CL9 - Avant processus de mise en forme galénique** | **CL9 - après mise en forme comprimés** |
|---|---|---|---|---|---|---|
| 30minutes | 1% | 15% | 16% | 28% | 39% | 66% |
| 60minutes | 3% | 22% | 28% | 44% | 67% | 90% |
| 120minutes | 7% | 27% | 48% | 63% | > 90% | > 90% |
| 180minutes | 11% | 30% | 63% | 74% | > 90% | > 90% |
| 240minutes | 16% | 32% | 74% | 82% | > 90% | > 90% |
| 300minutes | 21% | 34% | 82% | 88% | > 90% | > 90% |
| 360minutes | 26% | 35% | 88% | > 90 % | > 90% | > 90% |

Le tableau ci-dessous mentionne les valeurs des écarts temps à temps et les taux de variation à 180 minutes et au-delà :

**Tableau 12**

| **Identification de la composition // Temps de prélèvement** | **CL7 - Ecart temps à temps** | **CL 7 - Taux de variation** | **CL8 - Ecart temps à temps** | **CL 8 - Taux de variation** | **CL9 - Ecart temps à temps** | **CL9** - **Taux de variation** |
|---|---|---|---|---|---|---|
| 30minutes | 13% | | 12% | | 27% | |
| 60minutes | 19% | | 16% | | 22% | |
| 120minutes | 20% | | 15% | | N/A | |
| 180minutes | 19% | > 100% | 11% | 18% | N/A | N/A |
| 240minutes | 16% | > 100% | 8% | 11% | N/A | N/A |
| 300minutes | 13% | 61% | 5% | 6% | N/A | N/A |
| 360minutes | 9% | 36% | N/A | N/A | N/A | N/A |

Sur cet exemple, les compositions CL8 et CL9 permettent une amélioration de la résistance à un stress mécanique. Via la composition CL7, on observe qu'une quantité minimale de composant B est requis.

## Revendications

1. Composition lipidique comprenant pour 100% de sa masse :
- de 40% à 99,9% massique d'un ensemble comprenant pour 100% de sa masse de 90% à 100% massique de cire d'abeille et jusqu'à 10% massique d'au moins un autre excipient lipidique,
- de 0,1% à 60% massique d'au moins un tensioactif lipophile choisi parmi les esters de sorbitan.

2. Composition lipidique selon la revendication 1, **caractérisée en ce que** l'excipient lipidique est choisi parmi les cires animales, les cires végétales, les cires minérales, les cires synthétiques ou les huiles végétales hydrogénées.

3. Composition lipidique selon l'une des revendications 1 ou 2, **caractérisée en ce que** le tensioactif lipophile est un tensioactif lipophile de la famille des esters de sorbitan, plus particulièrement un élément du groupe constitué par le monolaurate de sorbitan, le monopalmitate de sorbitan, le monostéarate de sorbitan et le monooléate de sorbitan, et encore plus particulièrement le monopalmitate de sorbitan ou le monostéarate de sorbitan, et encore plus particulièrement le monostéarate de sorbitan.

4. Composition lipidique selon l'une des revendications 1 à 3, **caractérisée en ce que** ladite composition comprend de 0 à 20% d'un ou plusieurs tensioactifs hydrophiles.

5. Composition lipidique selon la revendication 4, **caractérisée en ce que** le ou les tensioactifs hydrophiles sont choisis parmi la lécithine de soja, les esters de sorbitan éthoxylés, les alcools polyéthoxylés, les acides polyéthoxylés, les esters de polyglycérol, les éthers de glucose et les copolymères blocs d'oxydes d'éthylène et de propylène.

6. Composition lipidique selon la revendication 4, **caractérisée en ce que** le tensioactif hydrophobe est un ester de sorbitan et le tensioactif hydrophile est choisi parmi les esters de sorbitan polyéthoxylés.

7. Composition lipidique selon la revendication 6, **caractérisée en ce que** ladite composition comprend :
- 75% de cire d'abeille,
- 20% de stéarate de sorbitan, et
- 5% d'oléate de sorbitan polyéthoxylé à 20 moles d'oxyde d'éthylène (ou polysorbate 80).

8. Composition lipidique selon la revendication 6, **caractérisée en ce que** ladite composition comprend :
- 50% de cire d'abeille,
- 45% de stéarate de sorbitan, et
- 5% d'oléate de sorbitan polyéthoxylé à 20 moles d'oxyde d'éthylène (ou polysorbate 80).

9. Composition lipidique selon l'une des revendications 1 à 6, **caractérisée en ce que** ladite composition comprend de 0 à 20% d'au moins un adjuvant d'enrobage.

10. Composition lipidique selon la revendication 7, **caractérisée en ce que** le ou les adjuvants d'enrobage sont choisis parmi les diluants, les arômes, les appétants, les colorants, les antioxydants, les agents plastifiants, les anti-mousses et les désintégrants.

11. Composition (CA) à libération contrôlée comprenant :
- au moins une composition telle que définie à l'une des revendications 1 à 10, et
- au moins une substance active pharmaceutique, prophylactique ou alimentaire.

12. Composition (CA) selon la revendication 11, **caractérisée en ce qu'**elle est destinée à être administrée oralement chez l'être humain ou animal.

13. Composition (CA) selon l'une des revendications 11 ou 12, **caractérisée en ce qu'**elle est de forme solide.

14. Utilisation d'une composition lipidique telle que définie à l'une des revendications 1 à 10 pour l'encapsulation d'une substance active pharmaceutique, thérapeutique, prophylactique, ou alimentaire pour l'être humain ou animal dans une formulation galénique.

15. Procédé de fabrication d'une formulation galénique comprenant une composition (CA) selon l'une revendications 11 à 13, comprenant au moins :
a) une étape de préparation de la composition lipidique telle que définie à l'une des revendications 1 à 11,
b) une étape de mélange et d'encapsulation d'une substance active pharmaceutique, prophylactique ou alimentaire avec la composition lipidique préparée à l'étape a), de manière obtenir une composition (C_{A}) selon l'une des revendications 11 à 13, et
c) une étape de mise en forme galénique de la composition (C_{A}) préparée à l'étape b), impliquant un stress mécanique.

16. Procédé de fabrication selon la revendication 15, **caractérisé en ce que** l'étape b) de mélange et d'encapsulation comprend :
- une première sous-étape de chauffage de la composition lipidique préparée à l'étape a) à une température supérieure de 10 à 15°C de la plus haute température de fusion des différents ingrédients de ladite composition lipidique de manière à faire fondre ladite composition lipidique,
- une deuxième sous-étape de mélange de la composition lipidique fondue avec la substance active sous forme dispersée ou fondue, et
- une troisième sous-étape de pulvérisation de la composition obtenue à la deuxième sous-étape dans un air ambiant ou refroidi ou dans un liquide refroidi afin d'obtenir des particules solidifiées de composition (C_{A}).

17. Procédé de fabrication selon l'une des revendications 15 ou 16, **caractérisé en ce que** l'étape de mise en forme galénique impliquant un stress mécanique est choisie parmi la compression, la mise en gélules, le compactage, la mise en emballage, la mise en sachets, la mise en sticks, l'extrusion, la granulation, la pelletisation.

## Patentansprüche

1. Eine Lipidzusammensetzung, umfassend für 100% ihrer Masse:
- von 40 Masse-% bis 99,9 Masse-% einer Gesamtheit, umfassend für 100% ihrer Masse von 90 Masse-% bis 100 Masse-% Bienenwachs und bis zu 10 Masse-% mindestens eines anderen Lipidhilfsstoffs,
- von 0,1 Masse-% bis 60 Masse-% mindestens eines lipophilen Tensids, ausgewählt aus Sorbitanestern.

2. Die Lipidzusammensetzung nach Anspruch 1, **dadurch gekennzeichnet, dass** der Lipidhilfsstoff ausgewählt ist aus tierischen Wachsen, pflanzlichen Wachsen, mineralischen Wachsen, synthetischen Wachsen oder hydrierten Pflanzenölen.

3. Die Lipidzusammensetzung nach einem der Ansprüche 1 oder 2, **dadurch gekennzeichnet, dass** das lipophile Tensid ein lipophiles Tensid aus der Familie der Sorbitanester ist, insbesondere ein Element der Gruppe bestehend aus Sorbitanmonolaurat, Sorbitanmonopalmitat, Sorbitanmonostearat und Sorbitanmonooleat, und noch spezieller Sorbitanmonopalmitat oder Sorbitanmonostearat, und noch spezieller Sorbitanmonostearat.

4. Die Lipidzusammensetzung nach einem der Ansprüche 1 bis 3, **dadurch gekennzeichnet, dass** die besagte Zusammensetzung 0 bis 20 % eines oder mehrerer hydrophiler Tenside umfasst.

5. Die Lipidzusammensetzung nach Anspruch 4, **dadurch gekennzeichnet, dass** das oder die hydrophilen Tenside ausgewählt sind aus Sojalecithin, ethoxylierten Sorbitanestern, polyethoxylierten Alkoholen, polyethoxylierten Säuren, Polyglycerinestern, Glucoseethern und Blockcopolymeren von Ethylenoxid und Propylenoxid.

6. Die Lipidzusammensetzung nach Anspruch 4, **dadurch gekennzeichnet, dass** das hydrophobe Tensid ein Sorbitanester ist und das hydrophile Tensid ausgewählt ist aus polyethoxylierten Sorbitanestern.

7. Die Lipidzusammensetzung nach Anspruch 6, **dadurch gekennzeichnet, dass** die besagte Zusammensetzung umfasst:
- 75% Bienenwachs,
- 20% Sorbitanstearat, und
- 5% polyethoxyliertes Sorbitanoleat mit 20 Mol Ethylenoxid (oder Polysorbat 80).

8. Die Lipidzusammensetzung nach Anspruch 6, **dadurch gekennzeichnet, dass** die besagte Zusammensetzung umfasst:
- 50% Bienenwachs,
- 45% Sorbitanstearat, und
- 5% polyethoxyliertes Sorbitanoleat mit 20 Mol Ethylenoxid (oder Polysorbat 80).

9. Die Lipidzusammensetzung nach einem der Ansprüche 1 bis 6, **dadurch gekennzeichnet, dass** die besagte Zusammensetzung 0 bis 20 % mindestens eines Überzugshilfsstoffs umfasst.

10. Die Lipidzusammensetzung nach Anspruch 7, **dadurch gekennzeichnet, dass** der oder die Überzugshilfsstoffe ausgewählt sind aus Verdünnungsmitteln, Aromen, Appetitanregern, Farbstoffen, Antioxidantien, Weichmachern, Antischaummitteln und Sprengmitteln.

11. Eine Zusammensetzung (CA) mit kontrollierter Freisetzung, umfassend:
- mindestens eine Zusammensetzung wie in einem der Ansprüche 1 bis 10 definiert, und
- mindestens einen pharmazeutischen, prophylaktischen oder Nahrungsmittelwirkstoff.

12. Die Zusammensetzung (CA) nach Anspruch 11, **dadurch gekennzeichnet, dass** sie zur oralen Verabreichung bei Menschen oder Tieren bestimmt ist.

13. Die Zusammensetzung (CA) nach einem der Ansprüche 11 oder 12, **dadurch gekennzeichnet, dass** sie in fester Form vorliegt.

14. Verwendung einer Lipidzusammensetzung wie in einem der Ansprüche 1 bis 10 definiert zur Verkapselung eines pharmazeutischen, therapeutischen, prophylaktischen oder Nahrungsmittelwirkstoffs für Menschen oder Tiere in einer galenischen Formulierung.

15. Ein Verfahren zur Herstellung einer galenischen Formulierung, die eine Zusammensetzung (CA) nach einem der Ansprüche 11 bis 13 umfasst, umfassend mindestens:
a) einen Schritt der Herstellung der Lipidzusammensetzung wie in einem der Ansprüche 1 bis 11 definiert,
b) einen Schritt des Mischens und Verkapselns eines pharmazeutischen, prophylaktischen oder Nahrungsmittelwirkstoffs mit der in Schritt a) hergestellten Lipidzusammensetzung, um eine Zusammensetzung (CA) nach einem der Ansprüche 11 bis 13 zu erhalten, und
c) einen Schritt der galenischen Formgebung der in Schritt b) hergestellten Zusammensetzung (CA), der eine mechanische Belastung beinhaltet.

16. Das Herstellungsverfahren nach Anspruch 15, **dadurch gekennzeichnet, dass** der Schritt b) des Mischens und Verkapselns umfasst:
- einen ersten Teilschritt des Erhitzens der in Schritt a) hergestellten Lipidzusammensetzung auf eine Temperatur, die 10 bis 15 °C über der höchsten Schmelztemperatur der verschiedenen Bestandteile der besagten Lipidzusammensetzung liegt, um die besagte Lipidzusammensetzung zu schmelzen,
- einen zweiten Teilschritt des Mischens der geschmolzenen Lipidzusammensetzung mit dem Wirkstoff in dispergierter oder geschmolzener Form, und
- einen dritten Teilschritt des Versprühens der im zweiten Teilschritt erhaltenen Zusammensetzung in Umgebungs- oder gekühlter Luft oder in einer gekühlten Flüssigkeit, um verfestigte Partikel der Zusammensetzung (CA) zu erhalten.

17. Das Herstellungsverfahren nach einem der Ansprüche 15 oder 16, **dadurch gekennzeichnet, dass** der Schritt der galenischen Formgebung, der eine mechanische Belastung beinhaltet, ausgewählt ist aus Kompression, Abfüllen in Kapseln, Kompaktierung, Verpacken, Abfüllen in Beutel, Abfüllen in Sticks, Extrusion, Granulierung, Pelletierung.

## Claims

1. A lipid composition comprising for 100% of its mass:
- from 40% to 99.9% by mass of a set comprising for 100% of its mass from 90% to 100% by mass of beeswax and up to 10% by mass of at least one other lipid excipient,
- from 0.1% to 60% by mass of at least one lipophilic surfactant chosen from sorbitan esters.

2. The lipid composition according to claim 1, **characterized in that** the lipid excipient is chosen from animal waxes, vegetable waxes, mineral waxes, synthetic waxes or hydrogenated vegetable oils.

3. The lipid composition according to one of claims 1 or 2, **characterized in that** the lipophilic surfactant is a lipophilic surfactant of the sorbitan ester family, more particularly an element of the group consisting of sorbitan monolaurate, sorbitan monopalmitate, sorbitan monostearate and sorbitan monooleate, and even more particularly sorbitan monopalmitate or sorbitan monostearate, and even more particularly sorbitan monostearate.

4. The lipid composition according to one of claims 1 to 3, **characterized in that** said composition comprises from 0 to 20% of one or more hydrophilic surfactants.

5. The lipid composition according to claim 4, **characterized in that** the hydrophilic surfactant or surfactants are chosen from soy lecithin, ethoxylated sorbitan esters, polyethoxylated alcohols, polyethoxylated acids, polyglycerol esters, glucose ethers and block copolymers of ethylene oxide and propylene oxide.

6. The lipid composition according to claim 4, **characterized in that** the hydrophobic surfactant is a sorbitan ester and the hydrophilic surfactant is chosen from polyethoxylated sorbitan esters.

7. The lipid composition according to claim 6, **characterized in that** said composition comprises:
- 75% beeswax,
- 20% sorbitan stearate, and
- 5% polyethoxylated sorbitan oleate with 20 moles of ethylene oxide (or polysorbate 80).

8. The lipid composition according to claim 6, **characterized in that** said composition comprises:
- 50% beeswax,
- 45% sorbitan stearate, and
- 5% polyethoxylated sorbitan oleate with 20 moles of ethylene oxide (or polysorbate 80).

9. The lipid composition according to one of claims 1 to 6, **characterized in that** said composition comprises from 0 to 20% of at least one coating adjuvant.

10. The lipid composition according to claim 7, **characterized in that** the coating adjuvant or adjuvants are chosen from diluents, flavors, palatability agents, colorants, antioxidants, plasticizing agents, anti-foaming agents and disintegrants.

11. A controlled-release composition (CA) comprising:
- at least one composition as defined in one of claims 1 to 10, and
- at least one pharmaceutical, prophylactic or food active substance.

12. The composition (CA) according to claim 11, **characterized in that** it is intended for oral administration in humans or animals.

13. The composition (CA) according to one of claims 11 or 12, **characterized in that** it is in solid form.

14. Use of a lipid composition as defined in one of claims 1 to 10 for the encapsulation of a pharmaceutical, therapeutic, prophylactic, or food active substance for humans or animals in a galenic formulation.

15. A method of manufacturing a galenic formulation comprising a composition (CA) according to one of claims 11 to 13, comprising at least:
a) a step of preparing the lipid composition as defined in one of claims 1 to 11,
b) a step of mixing and encapsulating a pharmaceutical, prophylactic or food active substance with the lipid composition prepared in step a), in order to obtain a composition (CA) according to one of claims 11 to 13, and
c) a step of galenic shaping of the composition (CA) prepared in step b), involving mechanical stress.

16. The manufacturing method according to claim 15, **characterized in that** step b) of mixing and encapsulation comprises:
- a first sub-step of heating the lipid composition prepared in step a) to a temperature 10 to 15°C higher than the highest melting temperature of the different ingredients of said lipid composition so as to melt said lipid composition,
- a second sub-step of mixing the molten lipid composition with the active substance in dispersed or molten form, and
- a third sub-step of spraying the composition obtained in the second sub-step in ambient or cooled air or in a cooled liquid in order to obtain solidified particles of composition (CA).

17. The manufacturing method according to one of claims 15 or 16, **characterized in that** the galenic shaping step involving mechanical stress is chosen from compression, filling into capsules, compaction, packaging, filling into sachets, filling into sticks, extrusion, granulation, pelletization.
